Europäisches Patentamt

European Patent Office

Office européen des brevets

(11) Publication number: 0 300 724
A1

## (12) EUROPEAN PATENT APPLICATION

(21) Application number: 88306580.7

(51) Int. Cl.4: C07C 19/08

(22) Date of filing: 19.07.88

The title of the invention has been amended (Guidelines for Examination in the EPO, A-III, 7.3).

(30) Priority: 24.07.87 GB 8717595

(43) Date of publication of application:
25.01.89 Bulletin 89/04

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: McCulloch, Archibald
Barrymore Marbury Road Comberbach
Northwich Cheshire CW9 6A(GB)
Inventor: Powell, Richard Llewellyn
9 Sadlers Wells Bunbury
Tarporley Cheshire(GB)
Inventor: Young, Brian David
31 Ludlow Close
Winsford Cheshire(GB)

(74) Representative: Stephenson, Kenneth et al
Imperial Chemical Industries PLC Legal
Department: Patents PO Box 6 Bessemer
Road
Welwyn Garden City Herts, AL7 1HD(GB)

(54) Process for the preparation of 1,1,1,2-tetrafluoroethane.

(57) A method for the preparation of 1,1,1,2-tetrafluoroethane which comprises contacting a tetrahalogenoethane or trihalogenoethylene in which at least one of the halogen atoms is not fluorine or an unsymmetrical dihalogenoethylene with an antimony pentahalide of the formula:
$SbCl_xF_y$
wherein the sum of x and y is 5 and the value of y is in the range 3 to 5, and optionally with a corresponding antimony trihalide, at a temperature in the range 50-150°C

Xerox Copy Centre

# CHEMICAL PROCESS

This invention relates to a chemical process and more particularly to a process for the manufacture of 1,1,1,2-tetrafluoroethane.

It is already known to manufacture chlorofluorocarbons and chlorofluorohydrocarbons by reacting chlorocarbons and chlorohydrocarbons with antimony halides and hydrogen fluoride, the antimony halides being of the general type $SbCl_xF_y$ wherein the sum of x and y is approximately 5, some trihalide possibly being present. Examples of this reaction, known as the Swarts reaction, include the production of chlorodifluoromethane from chloroform and the production of trichlorofluoromethane and dichlorodifluoromethane from carbon tetrachloride.

The Swarts reaction has not hitherto been regarded as suitable for the manufacture of all fluorohydrocarbons. Thus, other methods have been proposed for the manufacture of 1,1,1,2-tetrafluoroethane which is a useful refrigerant, aerosol propellant, blowing agent and solvent. For example, our United Kingdom Patent No 1578933 describes a process for the manufacture of tetrafluoroethanes by hydrogenating dichlorotetrafluoroethanes at elevated temperatures, for example temperatures in the range 200-450° C. However, dichlorotetrafluoroethanes exist industrially as a mixture of two difficulty separable isomers, only one of which is converted to 1,1,1 2-tetrafluoroethane. Also, our United Kingdom Patent No. 2004539 describes the manufacture of 1,1,1,2-tetrafluoroethane by reacting trifluoroethylene in the vapour phase with hydrogen fluoride in the presence of chromium oxide, suitable reaction temperatures being in the range 200-500° C. Both of these methods for the production of tetrafluoroethane are expensive to operate.

It has now been found that 1,1,1,2-tetrafluoroethane can be obtained from various halohydrocarbons under mild temperature conditions by the action of appropriate antimony halides.

Thus, according to the invention, there is provided a method for the preparation of 1,1,1,2-tetrafluoroethane which comprises contacting a tetrahalogenoethane or trihalogenoethylene in which at least one of the halogen atoms is not fluorine or an unsymmetrical dihalogenoethylene with an antimony pentahalide of the formula:
$SbCl_xF_y$
wherein the sum of x and y is 5 and the value of y is in the range 3 to 5, and optionally with a corresponding antimony trihalide, at a temperature in the range 50-150° C.

Tetrahalogenoethanes which may be employed as starting materials in the method of the invention include both the 1,1,2,2- and 1,1,1,2-tetrahalogeno compounds provided that at least one halogen atom is not fluorine. It is preferred that each non-fluorine halogen atom is chlorine. Thus, suitable tetrahalogenoethanes include the various tetrachloroethane, trichlorofluoroethane, dichlorodifluoroethane and chlorotrifluoroethane isomers, especially 1-chloro-2,2,2-trifluoroethane. Trihalogenoethylenes which may be used include trichloroethylene and unsymmetrical dihalogenoethylenes include 1,1-dichloroethylene.

In operating the method of the invention, the halogenated ethylene or halogenated ethane is contacted with the antimony halide at the operating temperature. During the reaction, the chloride content of the antimony halide increases and refluorination may be effected by contacting the used antimony halide with hydrogen fluoride. In practice, the starting material (halogenated ethylene or halogenated ethane) and hydrogen fluoride may be contacted simultaneously with the antimony halide. Alternatively, the antimony halide may be contacted successively with the starting material and the hydrogen fluoride. The 1,1,1,2-tetrafluoroethane may be separated from any by-products using conventional techniques.

The invention is illustrated but not limited by the following Example.

## EXAMPLE 1

A 125 ml Hasteloy lined autoclave was charged with 70 g (0.32 m) $SbF_5$ and 2 g (0.007 m) $SbCl_5$ and assembled. After pressure testing to 400 psig the autoclave was cooled to -60° C and evacuated. 24 g $CF_3CH_2Cl$ (0.2 m) was then vacuum distilled into the autoclave and after warming slowly to room temperature, the autoclave was heated with stirring to 70° C. After 2 hours at 70° C, the autoclave was cooled and a gas sample taken from the autoclave. GC analysis of this sample showed 4.7% $CF_3CH_2F$ had been formed. After heating at 100° C for a further 2 hours, another gas sample was taken and was found to contain 24.5% $CF_3CH_2F$. A gas sample taken after heating for a further 2 hours at 125° C was found to contain 52.8% $CF_3CH_2F$. However, the gas sample taken after heating for a further 2 hours at 150° C was found to contain only 26.5% $CF_3CH_2F$, the major product being $CF_3CF_2H$ (33.4%).

The autoclave was then heated to 40° C and the remaining gases were vacuum distilled into a stainless steel cylinder. GC analysis of the liquid showed it to contain 20.9% $CF_3CH_2F$.

GC analysis performed on Varian 3400 gas chromatograph on a 6′ x $\frac{1}{8}$″ PPQ packed column.

## EXAMPLE 2

A 125 ml Hasteloy lined autoclave was charged with 58.9g (0.27 mol) of $SbF_5$ and assembled. After pressure testing to 400 psig, the autoclave was cooled in liquid nitrogen and evacuated. 32.3g of $CF_3CH_2Cl$ (0.27 mol) was then vacuum distilled into the autoclave and after warming slowly to room temperature the autoclave was heated with stirring to 125° C. After 2 hours at 125° C, the autoclave was cooled and a gas sample taken. GC analysis of this sample showed that it contained 45.1% $CF_3CH_2F$. After heating at 125° C for a further two hours, the autoclave was again cooled and a second gas sample taken. This was found to contain 42.9% $CF_3CH_2F$. The composition of vapour samples taken after six hours and eight hours at 125° c was not significantly different. The final composition of the vapour sample was $CF_3CH_2F$ (42.0%), $CF_3CHF_2$ (10.0%), $CF_3CHClF$ (18.8%), $CF_3CH_2Cl$ (29.2%). The remaining gases were vacuum distilled into a stainless steel cylinder. G.C. analysis of the liquid showed it to contain 29.4% to $CF_3CH_2F$. G.C. analysis performed on Varian 3400 gas chromatograph on a 6′ x $\frac{1}{4}$″ PPQ packed column.

## EXAMPLE 3

A 125ml Hasteloy lined autoclave was charged with 109.0g of $SbF_5$ (0.5 mol) aud 3.0g of $SbCl_5$ - (0.01 mol) and assembled. After pressure testing to 400 psig, the autoclave was cooled to -40° C and evacuated. 16.8g of $CCl_3CH_2Cl$ (0.1) mol was then transferred into the autoclave by suction. The autoclave was then cooled to -70° C and re-evacuated. After warming to room temperature, the autoclave was heated with stirring to 80° C and maintained at this temperature for two hours. The autoclave was then cooled and a gas sample taken. G. C. analysis showed that 1.4% $CF_3CH_2F$, had been formed. 69.1% of $CF_3CH_2Cl$ was also present in this sample. After heating for a further 2 hours, at 100° C, the autoclave was again cooled and a gas sample taken. G.C. analysis showed this to contain 5.4% $CF_3CH_2F$, 63.2% $CF_3CH_2Cl$, 22.6% $CF_3CHClF$, 3.3% $CF_3CHF_2$.

G.C. analysis performed on Varian 3400 gas chromatograph on a 6′ x $\frac{1}{4}$″ PPQ packed column.

## Claims

1. A method for preparation of 1,1,1,2-tetrafluoroethane which comprises contacting a tetrahalogenoethane or trihalogenoethylene in which at least one of the halogen atoms is not fluorine on at unsymmetrical dihalogenoethylene with an antimony pentahalide of the formula: $SbCl_xF_y$ wherein the sum of x and y is 5 and the value of y is in the range 3 to 5, and optionally with a corresponding antimony trihalide, at a temperature in the range 50-150° C.

2. A method according to claim 1 wherein the tetrahalogenoethane is 1-chloro - 2,2,2,-trifluoroethane or 1,1,1,2- tetrachloroethane.

European. Patent Office

**PARTIAL EUROPEAN SEARCH REPORT**

which under Rule 45 of the European Patent Convention
shall be considered, for the purposes of subsequent
proceedings, as the European search report

Application number

EP 88 30 6580

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB-A- 881 003 (I.C.I.) | | C 07 C 19/08 |
| A | US-A-4 311 863 (GUMPRECHT) | | |
| A | US-A-2 490 764 (A.F. BENNING et al.) | | |
| A | US-A-3 240 826 (R.A. DAVIS) | | |
| A | US-A-2 426 172 (A.F. BENNING) | | |
| | ------------ | | |

TECHNICAL FIELDS
SEARCHED (Int. Cl.4)

C 07 C 19/00

C 07 C 17/00

## INCOMPLETE SEARCH

The Search Division considers that the present European patent application does not comply with
the provisions of the European Patent Convention to such an extent that it is not possible to carry
out a meaningful search into the state of the art on the basis of some of the claims.
Claims searched completely: all
Claims searched incompletely:
Claims not searched:
Reason for the limitation of the search:

The last words of line 4 of claim 1 have been
read - in accordance with the corresponding
passage on page 2 - as: "....not fluorine or
an"

| Place of search | Date of completion of the search | Examiner |
|---|---|---|
| The Hague | 13-10-1988 | VAN GEYT |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1505.1. 03.82